## Europäisches Patentamt

## European Patent Office

## Office européen des brevets

(19)

(11) Veröffentlichungsnummer: **0 043 520**
**B1**

(12) **EUROPÄISCHE PATENTSCHRIFT**

(45) Veröffentlichungstag der Patentschrift:
21.09.83

(21) Anmeldenummer: 81104966.7

(22) Anmeldetag: 26.06.81

(51) Int. Cl.³: **C 07 C 89/00**, C 07 C 91/44

(54) Verfahren zur Herstellung von 4-Amino-3-methyl-phenol.

(30) Priorität: 08.07.80 DE 3025805

(43) Veröffentlichungstag der Anmeldung:
13.01.82 Patentblatt 82/2

(45) Bekanntmachung des Hinweises auf die Patenterteilung:
21.09.83 Patentblatt 83/38

(84) Benannte Vertragsstaaten:
BE CH DE FR GB IT LI

(73) Patentinhaber: BAYER AG, Zentralbereich Patente,
Marken und Lizenzen, D-5090 Leverkusen 1, Bayerwerk
(DE)

(72) Erfinder: Skipka, Guido, Dr., Theodor-Heuss-Strasse 22,
D-5300 Bonn 2 (DE)
Erfinder: Alles, Hans-Ulrich, Dr.,
Reiner-Huetten-Strasse 15, D-5068 Odenthal (DE)
Erfinder: Randau, Gert, Dr., Heidberger Strasse 57,
D-5068 Odenthal (DE)
Erfinder: Niese, Manfred, Dr.,
Elisabeth-Langgaesser-Strasse 29, D-5090 Leverkusen
(DE)

(56) Entgegenhaltungen:
CH-A- 391 724
DE-A-2 256 003

CHEMICAL ABSTRACTS, Band 78, Nr. 17, 30. April
1973, Seite 437, Nr. 110738q Columbus, Ohio, U.S.A. E.
YU. BELYAEV et al.: "Structure and properties of nitroso compounds. Reduction of nitroso compounds by
hydrazine hydrate"

JOURNAL OF ORG. CHEMISTRY, Band 16, 1947, Seiten 405-421 Washington, D.C., U.S.A., R.C. ELDERFIELD
et al.: "Synthesis of BZ-substituted quinazolines and
antimalarials from them. A contribution to the chemistry of quinazoline"

(56) Entgegenhaltungen:
ORGANIC SYNTHESES, collective volume 1, John Wiley & Sons, Seiten 511-513 London, G.B., E. KREMERS
et al.: "Thymoquinone" Seite 512 (B)

Verfahren zur Herstellung von 4-Amino-3-methyl-phenol

Die Erfindung betrifft ein Verfahren zur Herstellung von 4-Amino-3-methyl-phenol aus 4-Nitroso-3-methyl-phenol.

Es ist bekannt, 4-Nitroso-3-methyl-phenol in wässrigem Ammoniak zu lösen und mit Schwefelwasserstoff zu 4-Amino-3-methyl-phenol zu reduzieren [J. org. Chem. 12, 417 (1947)].

Nachteilig bei diesem Verfahren ist das Arbeiten in Gegenwart von Schwefelwasserstoff, da wegen der Giftigkeit des Schwefelwasserstoffs aufwendige Sicherheitsvorkehrungen getroffen werden müssen, was die Wirtschaftlichkeit des Verfahrens beeinträchtigt.

Weiterhin wird in der schweizerischen Patentschrift 391 724 ein allgemeines Verfahren zur Reduktion aromatischer Nitro- oder Nitrosoverbindungen zu Aminen beschrieben, das dadurch gekennzeichnet ist, dass man die Reduktion mittels wässriger Lösungen oder Suspensionen von ein Pyridin enthaltenden Fe II-Komplexverbindungen durchführt.

Für das in der schweizerischen Patentschrift 391 724 beschriebene Verfahren ist es zur Vermeidung von unerwünschten Nebenprodukten, die bei dem bekannten Béchamp-Verfahren im allgemeinen erhalten werden, und zur Erhöhung der Ausbeute an Aminen wesentlich, dass die Reduktion in Gegenwart von Pyridin enthaltenden Fe II-Komplexverbindungen durchgeführt wird. Nachteilig bei diesem Verfahren ist die Anwesenheit von Pyridin bzw. Pyridineisenverbindungen, die von dem gewünschten Amin in technisch aufwendiger Weise abgetrennt werden müssen.

Es wurde nun ein Verfahren zur Herstellung von 4-Amino-3-methyl-phenol aus 4-Nitroso-3-methyl-phenol gefunden, das dadurch gekennzeichnet ist, dass man 4-Nitroso-3-methyl-phenol mit Eisen in saurem wässrigem Medium bei Temperaturen von 20 bis 100°C und in einem pH-Bereich von 2 bis 6,5 umsetzt.

Die erfindungsgemässe Umsetzung wird im allgemeinen mit der 1,2- bis 10fachen äquivalenten Menge Eisen, bevorzugt mit der 2- bis 4fachen äquivalenten Menge Eisen, durchgeführt.

Das Eisen wird im allgemeinen in Form von Eisenspänen in das erfindungsgemässe Verfahren eingesetzt. Es können Eisenspäne unterschiedlicher Qualität und Korngrösse verwendet werden. Bevorzugt verwendet man gesiebte Eisenspäne mit geringem Staubanteil.

Das Eisen wird üblicherweise auf einmal vor Beginn der Umsetzung dem Reaktionsgemisch zugesetzt.

Das erfindungsgemässe Verfahren wird im allgemeinen bei Temperaturen von 20 bis 100°C, bevorzugt bei 30 bis 50°C, durchgeführt.

Üblicherweise führt man das erfindungsgemässe Verfahren in einem pH-Bereich von 2 bis 6,5, bevorzugt in einem Bereich von 5 bis 6, besonders bevorzugt in einem pH-Bereich von 5,2 bis 5,9, durch.

Zur Herstellung des wässrig-sauren Mediums, in dem die erfindungsgemässe Umsetzung durchgeführt wird, können verschiedene anorganische oder organische Säuren, wie Salzsäure, Schwefelsäure oder aliphatische Carbonsäuren mit 2 bis 7 Kohlenstoffatomen, bevorzugt 2 bis 5 Kohlenstoffatomen, eingesetzt werden. Zum Beispiel werden als aliphatische Carbonsäuren genannt: Essigsäure, Propionsäure und Buttersäure. Bevorzugt wird Essigsäure in das erfindungsgemässe Verfahren eingesetzt.

Das erfindungsgemässe Verfahren wird im allgemeinen so durchgeführt, dass man die Eisenspäne in einem Gemisch aus Wasser und der entsprechenden Säure vorlegt, diese Suspension auf die entsprechende Reaktionstemperatur erwärmt und anschliessend das 4-Nitroso-3-methyl-phenol suspendiert in Wasser der sauren Eisenspänesuspension zusetzt.

Es ist auch möglich, das 4-Nitroso-3-methyl-phenol als Feststoff der sauren Eisenspäne-Suspension zuzugeben.

Das nach der Umsetzung erhaltene Eisenoxidhaltige Gemisch aus Wasser und 4-Amino-3-methyl-phenol wird nach dem Versetzen des Reaktionsgemisches mit Natriumsulfit und 50%iger Natronlauge von dem überschüssigen Eisen abgetrennt und nach dem Abscheiden des Eisenoxids weiter verarbeitet. Das nicht verbrauchte restliche Eisen wird um den Anteil des umgesetzten Eisens ergänzt und erneut für eine weitere Reduktion verwendet.

Das erfindungsgemässe Verfahren wird im allgemeinen bei Normaldruck durchgeführt. Es ist jedoch auch möglich, bei vermindertem oder erhöhtem Druck zu arbeiten.

Das erfindungsgemässe Verfahren kann sowohl diskontinuierlich als auch kontinuierlich durchgeführt werden.

Die Vorteile des erfindungsgemässen Verfahrens liegen in der einfachen Reaktionsdurchführung verbunden mit einer hohen Ausbeute und guten Qualität an 4-Amino-3-methyl-phenol.

Es ist überraschend, dass das erfindungsgemässe Verfahren so selektiv, d.h. ohne die Bildung von unerwünschten Neben- und Kondensationsprodukten, verläuft.

Weiterhin ist überraschend, dass die hohe Ausbeute und die ausserordentliche Selektivität des erfindungsgemässen Verfahrens zu erreichen sind, ohne den Zusatz von Nebenreaktionen verhindernden Pyridineisenverbindungen (vgl. hierzu die schweizerische Patentschrift 391 724, Seite 2, Zeilen 11–15).

Das 4-Amino-3-methyl-phenol dient als Zwischenprodukt für die Herstellung von Pflanzenschutzmitteln [vgl. DE-AS 1 145 162; CA 59, 9885d (1963)].

Beispiel

In einer Glasapparatur, bestehend aus einem 3-l-Fünfhals-Sulfonierbecher mit eingesetztem Balkenrührer aus rostfreiem Stahl, Innenthermometer und Tropftrichter mit Rührer wurden 600 g Eisenspäne mit 500 ml Wasser und 1 ml Essigsäure vorgelegt, wobei auf die Temperatur von 35°C erwärmt wurde.

Es wurden 196,5 g 4-Nitroso-3-methyl-phenol (75,8%ige Waren entsprechend 1,087 Mol) in 300 ml Wasser suspendiert. Die Suspension trug man innerhalb von 40 Minuten in die saure Eisenspäne-Suspension ein. Während des Eintragens der Suspension musste der Auslauf einer Probe auf Fliesspapier nach Tüpfeln mit 1 n Natronlauge hell bleiben. Im Verlauf der Reduktion wurden noch 4 ml Essigsäure zugegeben. Die Temperatur während der Reduktion wurde mit 800 g Eis auf 40°C gehalten. Man reduzierte noch 30 Minuten nach, versetzte den Ansatz mit 25 g Natriumsulfit und erwärmte auf 80°C. Nach Erreichen der Temperatur stellte man mit 85 ml 50%iger Natronlauge das Reaktionsgemisch auf den pH-Wert 12 ein und saugte die Suspension unverzüglich durch ein Filter auf 310 ml roher Salzsäure. Der Eisenoxid-Rückstand wurde mit etwas heissem Wasser gewaschen. Die klare Lösung wurde mit 35 g A-Kohle versetzt und bei einer Temperatur von 90°C filtriert. In 2300 ml Filtrat ermittelte man mit Hilfe einer Diazotierung 1,069 Mol entsprechend einer Ausbeute von 98,3% der Theorie.

## Patentansprüche

1. Verfahren zur Herstellung von 4-Amino-3-methyl-phenol aus 4-Nitroso-3-methyl-phenol, dadurch gekennzeichnet, dass man 4-Nitroso-3-methyl-phenol mit Eisen im sauren wässrigen Medium bei Temperaturen von 20 bis 100°C und in einem pH-Bereich von 2 bis 6,5 umsetzt.

2. Verfahren nach Anspruch 1, dadurch gekennzeichnet, dass man die Umsetzung bei Temperaturen von 30 bis 50°C durchführt.

3. Verfahren nach. Ansprüchen 1 und 2, dadurch gekennzeichnet, dass man die Umsetzung in einem pH-Bereich von 5 bis 6 durchführt.

4. Verfahren nach Ansprüchen 1 und 2, dadurch gekennzeichnet, dass man die Umsetzung in einem pH-Bereich von 5,2 bis 5,9 durchführt.

5. Verfahren nach Ansprüchen 1 bis 4, dadurch gekennzeichnet, dass man die Umsetzung mit der 1,2- bis 10fachen äquivalenten Menge Eisen durchführt.

6. Verfahren nach Ansprüchen 1 bis 4, dadurch gekennzeichnet, dass man die Umsetzung mit der 2- bis 4fachen äquivalenten Menge Eisen durchführt.

## Claims

1. Process for the preparation of 4-amino-3-methyl-phenol from 4-nitroso-3-methyl-phenol, characterised in that 4-nitroso-3-methyl-phenol is reacted with iron in an acid aqueous medium at temperatures from 20 to 100°C and in a pH range from 2 to 6.5.

2. Process according to Claim 1, characterised in that the reaction is carried out at temperatures from 30 to 50°C.

3. Process according to Claims 1 and 2, characterised in that the reaction is carried out in a pH range from 5 to 6.

4. Process according to Claims 1 and 2, characterised in that the reaction is carried out in a pH range from 5.2 to 5.9.

5. Process according to Claims 1 to 4, characterised in that the reaction is carried out with 1.2 to 10 times the equivalent amount of iron.

6. Process according to Claims 1 to 4, characterised in that the reaction is carried out with 2 to 4 times the equivalent amount of iron.

## Revendications

1. Procédé de production de 4-amino-3-méthylphénol à partir de 4-nitroso-3-méthylphénol, caractérisé en ce qu'on fait réagir du 4-nitroso-3-méthylphénol avec du fer en milieu aqueux acide à des températures de 20 à 100°C et dans une plage de pH de 2 à 5,5.

2. Procédé suivant la revendication 1, caractérisé en ce qu'on conduit la réaction à des températures de 30 à 50°C.

3. Procédé suivant les revendications 1 et 2, caractérisé en ce qu'on conduit la réaction dans une plage de pH de 5 à 6.

4. Procédé suivant les revendications 1 et 2, caractérisé en ce qu'on conduit la réaction dans une plage de pH de 5,2 à 5,9.

5. Procédé suivant les revendications 1 à 4, caractérisé en ce qu'on conduit la réaction avec la quantité 1,2 à 10 fois équivalente de fer.

6. Procédé suivant les revendications 1 à 4, caractérisé en ce qu'on conduit la réaction avec la quantité 2 à 4 fois équivalente de fer.